# EUROPÄISCHE PATENTSCHRIFT

(11) **EP 3 373 871 B1**
(45) Veröffentlichungstag und Bekanntmachung des Hinweises auf die Patenterteilung: **10.01.2024**
(21) Anmeldenummer: 16794663.1
(22) Anmeldetag: 14.11.2016
(51) Int. Cl.: A61F 6/22, A61B 17/12, A61F 6/24

(54) **UTEROTUBAR-IMPLANTAT-VORRICHTUNG**
UTEROTUBAR IMPLANT DEVICE
DISPOSITIF D'IMPLANT UTÉRO-TUBAIRE

(30) Priorität: 13.11.2015 DE 102015119639
(43) Veröffentlichungstag der Anmeldung: 19.09.2018
(73) Patentinhaber: Brohm-Schmitz-Rode, Andrea, 52070 Aachen (DE); Gerhards, Heinz, 52072 Aachen (DE)
(72) Erfinder: Brohm-Schmitz-Rode, Andrea, 52070 Aachen (DE)
(74) Vertreter: HGF
(86) Internationale Anmeldenummer: PCT/EP2016/077611
(87) Internationale Veröffentlichungsnummer: WO 2017/081329

(56) Entgegenhaltungen:
- US-A1- 2007 056 591
- US-A1- 2010 192 959
- US-A1- 2012 067 352
- US-A1- 2012 192 872
- US-A1- 2013 312 763
- US-B2- 8 322 341

## Beschreibung

Die vorliegende Erfindung betrifft eine Uterotubar-Implantat-Vorrichtung bzw. ein minimal-invasiv platzierbares Implantat für den Verschluss eines Eileiters zur Empfängnisverhütung.

Die Möglichkeit der Langzeitverhütung von Schwangerschaften (Kontrazeption) ist bei bestimmten individuellen sozialen und medizinischen Konstellationen hilfreich und wünschenswert. Es existieren verschiedene Verfahren zur Langzeitverhütung.

Zur Empfängnisverhütung sind Intrauterinpessare (IUP) bekannt, auch als "Spirale" bezeichnet. Dies sind Objekte, die in die Gebärmutter der Frau eingelegt werden und durch mechanischen Reiz der Gebärmutterschleimhaut, Milieuänderung oder Verdickung des Gebärmutterhalsschleims eine Schwangerschaft verhindern.

Es gibt mehrere Arten von empfängnisverhütenden Spiralen, die unterschieden werden, wobei die meisten davon aus Kunststoff ausgebildet sind.

Dies ist zum einen eine klassische Spirale mit einer Auflage aus einem Kupfer-Silberdraht oder auch Gold und zum anderen eine Spirale mit einem Hormon freisetzenden Kunststoffzylinder.

Kupferhaltige "Spiralen" sollen zusätzlich durch Absonderung kleinster Mengen Kupfer Spermien abtöten oder deaktivieren. IUP gelten als sehr sichere und langfristige Verhütungsmethoden. Ein Beispiel hierfür ist die Kupferspirale. Diese ist ein Kunststoffkörper, der Kupfer aufweist, nidationshemmend wirkt, und in die Gebärmutter eingebracht wird. Alternativ zur herkömmlichen Kupferspirale ist eine "Kupferkette" (GyneFix) bekannt. Dieses besteht aus an einem Faden aufgereihten Kupferzylindern und wird an der Gebärmutterwand fixiert, so dass ein Verrutschen oder Aussto-βen unwahrscheinlicher wird.

Die Kupfer-Silber-Spirale soll zusätzlich zum Wirkmechanismus des Kupferanteils eine durch den Silberanteil ausgelöste antibakterielle und fungizide Wirkung ausüben. Eine weitere Variante ist die Kupfer-Gold-Spirale. Sie hat einen Goldkern und wirkt im Wesentlichen wie die Kupfer-Silber-Spirale.

Alle Spiralen müssen, je nach Fabrikat, spätestens alle 3 bis 5 Jahre gewechselt werden.

Selbst beim richtigen Einlegen kann die Gebärmutterwand in seltenen Fällen aufgrund ihrer individuellen Beschaffenheit verletzt oder durchstoßen werden. Unmittelbar nach dem Einlegen kann es kurzfristig zu leichten Blutungen und Schmerzen sowie verlangsamtem Puls kommen. Eine Ausstoßung der Spirale, vor allem während einer starken Menstruation ist bei manchen Patientinnen möglich. Die Spirale kann infolge von Bewegungen der Gebärmuttermuskulatur ihre Lage verändern, selten auch in die Gebärmutterwand eindringen oder diese durchwandern. Dann muss sie operativ entfernt werden.

Eine Hormonspirale (Mirena^{®}) ist ein hormonell wirkendes (Gelbkörperhormonhaltiges) Langzeitverhütungsmittel, das in die Gebärmutter eingeführt wird. Durch die unmittelbare Nähe zum Wirkungsort kommt man mit einer sehr geringen Hormonmenge aus, wobei die wirksame Substanz einen ähnlichen Wirkmechanismus auslöst, wie die Gelbkörperhormon-Pille: der Zervikalschleim wird für Spermien undurchlässig. Sollte aber dennoch ein Spermium bis in den Eileiter gelangen, wird dort meist die Befruchtung verhindert. Und letztlich, sollte ein Ei dennoch befruchtet worden sein, wird die Einnistung in die Gebärmutterhöhle verhindert. Das Präparat muss spätestens alle 5 Jahre ausgetauscht werden.

Ein weiteres mögliches Verfahren ist der Verschluss beider Eileiter durch jeweils ein Implantat. Das Implantat kann hysteroskopisch oder durchleuchtungsgesteuert über einen dünnen Katheter im komprimierten Zustand in den Uterotubarwinkel und den proximalen Eileiter eingeführt und dort entfaltet werden. Nach Entfaltung soll das Implantat ortsstabil im proximalen Eileiter und Uterotubarwinkel verbleiben und in die Uterotubarschleimhaut einwachsen. Die Verschlusswirkung ist damit zunächst mechanisch. Durch eine medikamentöse Dotierung des Implantats oder von dessen Teilkomponenten kann eine zusätzliche pharmakologische Wirkung erzielt werden, solange das Medikament wirksam ist.

Im Rahmen der interventionellen Radiologie werden für die Embolisation von Gefä-βen kleine, transkatheteral applizierbare Ballons oder Metallspiralen verwendet. Oft reicht jedoch die mit einer Spirale erzielte Querschnittsverlegung nicht aus, um einen sicheren Verschluss zu produzieren. Der Nachteil des abwerfbaren Ballons besteht in einer zum Teil ungenügenden Fixation an der Gefäßwand, wodurch er nach Platzierung eine unerwünschte Lokalisation einnehmen kann.

Im Rahmen der transuterinen Tubensterilisation mit dem Hysteroskop sind bereits Tubenverschlüsse per Elektrokoagulation, durch Applikation von Gewebekleber und mit Hilfe von Ausgussstöpseln aus Silasticgummi beschrieben worden. Allen drei Verfahren ist gemeinsam, dass sie keinen sicheren Empfängnisschutz bieten und dass sie in der Regel nicht reversibel sind.

Aus der DE 91 09 006 U1 "Atherektomie-Angioplastie-Katheter" ist ein Katheter bekannt, der an der Spitze einen selbstexpandierbaren Korb aus einem geflochtenen tubulären Maschennetz besitzt. Der Maschenkorb ist bei diesem Gerät fest mit dem Katheter verbunden. Das System dient der Wiedereröffnung von Gefäßverschlüssen und wird nur für die Dauer der Behandlung in das Gefäßsystem eingebracht.

In der DE 92 05 797 U1 ist ein selbstexpandierbarer Maschenkorb für den Verschluss von menschlichen Hohlorganen beschrieben. Dieser Maschenkorb ist ein spindelförmig ausgebildeter Okklusionskörper, der als transuterin applizierbares intratubares Kontrazeptivum verwendbar ist. Dieser soll auch im Rahmen einer interventionellen Embolisationstherapie einen vaskulären Verschluss erzeugen können.

Ein solches Uterotubar-Implantat, welches einen beidseits geschlossenen Körper umfasst, der aus einem tubulären Drahtgeflecht ausgebildet ist, geht auch aus Schmitz-Rode T, Timmermans H, Uchida B, Kichikawa K, Nishida N, Günther RW, Rösch J. Self-expandable spindle for transcatheter vascular occlusion: in vivo experiments. Work in progress. Radiology. 1993 Jul;188(1):95-100, sowie aus Schmitz-Rode T, Ross PL, Timmermans H, Thurmond AS, Günther RW, Rösch J. Experimental nonsurgical female sterilization: transcervical implantation of microspindles in fallopian tubes. J Vase Interv Radiol. 1994 Nov-Dec;5(6):905-10, hervor.

Ein ähnliches Uterotubar-Implantat ist in der US 2005/0274384 A1 offenbart. Gemäß einer alternativen Ausführungsform können auch zwei getrennt voneinander in einer Längsrichtung hintereinander anordbare, gitterförmige, zylindrische selbstexpandierbare Körper vorgesehen sein, die an ihren distalen Enden konisch verjüngend ausgebildet sind.

In der US 2013/0220336 A1 ist ein Uterotubar-Implantat offenbart. Dieses Uterotubar-Implantat umfasst eine Metallwendel, die an einem distalen Ende mit einem zentral in der Wendel angeordneten Draht verbunden ist.

Aus der US 2013/0186409 A1 geht ein Implantat umfassend einen selbstexpandierenden Stent hervor. Diese Vorrichtung weist Streben auf, mittels derer der Eileiter durch "stretching" verschließbar sein soll.

In der US 6,634,361 B1 ist ein Okklusionskörper beschrieben, der einen zentralen Draht umfasst, auf dem zwei expandierbare spiralförmige Körper angeordnet sind.

Die US 7,975,697 B2 offenbart verschiedene Eileiter-Implantate.

Die US 7,987,853 B2 und die US 2013/0310683 A1 offenbaren Uterotubar-Implantate, die aus mehreren einzelnen Stents ausgebildet sind, die über Verbindungsmittel miteinander gekoppelt sind. Nach dem Einbringen der Stents werden die Verbindungsmittel entfernt.

Aus der US 8,235,047 B2 geht eine Verschlussvorrichtung hervor, die mehrere expandierbare Körper aufweist, die über ein mit diesen lösbar verbundenes Verbindungsmittel in den Körper einbringbar sind.

In der US 8,322,341 B2, die als nächstliegender Stand der Technik angesehen wird, ist ein Implantat zum Verschluss eines Körperlumens offenbart, das einen distal anordbaren ersten Körper umfasst, wobei nach der Anordnung des ersten Körpers ein zweiter, trichterförmiger Körper in Längsrichtung proximal zum ersten Körper einführbar und anordbar ist. Diese beiden Körper sind jedoch nicht miteinander gekoppelt, sondern verschiebbar zueinander ausgebildet.

In der US 2015/0201945 A1 ist eine Vorrichtung zum Verschließen eines Lumens, wie z.B. eines Eileiters, offenbart. Die Vorrichtung ist nicht selbstexpandierend und weist daher eine Betätigungsvorrichtung zum Expandieren auf. Weiterhin umfasst diese Vorrichtung einen Verschlusskörper, der aus longitudinalen Streben ausgebildet ist, die von einer impermeablen Hülle umgeben sind. Im distalen Bereich weist der Körper einen in etwa spindelförmigen Abschnitt auf. Im proximalen Bereich des Verschlusskörpers bilden die longitudinalen Streben einen im Wesentlichen zylindrischen Anker- bzw. Befestigungsabschnitt aus. Der Ankerabschnitt soll der Vorrichtung zusätzliche Stabilität verleihen, indem er die Fläche, mittels der die Vorrichtung in einem Lumen fixierbar ist, vergrößert. Am distalen Ende der Vorrichtung ist ein Betätigungsstrang der Betätigungseinrichtung befestigt, der sich durch den Abschnitt hindurch erstreckt. Durch Betätigung des Strangs ist, der rohrförmige Abschnitt expandierbar. Durch die Ausbildung des Körpers aus im Wesentlichen longitudinalen Streben weist die Vorrichtung eine hohe axiale Steifigkeit auf. Eine dadurch hervorgerufene unphysiologische axiale Streckung des gekrümmten Eileiters kann bei den Patientinnen zu Beschwerden führen.

Aus der DE 600 20 719 T2 geht ein Betätigungssystem für die Entfaltung eines Kontrazeptionsmittels im Eileiter hervor.

Aus der US 2010/192959 A1 geht eine transzervikale Verhütungsvorrichtung hervor (TCDs). Diese Vorrichtung kann z.B. drei in etwa spindelförmig ausgebildete und miteinander über Drahtabschnitte verbundene Körper umfassen. Neben einer Spindelform können die Körper auch andersartig ausgebildet sein. Hierbei ist vorgesehen, die Vorrichtung mittels eines Katheters im nicht expandierten Zustand an den Implantationsort einzubringen. Nach dem Entfernen des Katheters expandieren die Körper der Vorrichtung von selbst.

In der US 2007/056591 A1 sind eine Vorrichtung und ein Verfahren zum Verschluss des Eileiters offenbart. Diese Vorrichtung umfasst zwei Verankerungsmittel, die über ein langgestrecktes Verbindungsmittel miteinander verbunden sind. Dieses Verbindungsmittel kann z. B. als Stent oder gleichartige Struktur ausgebildet sein. Diesem Dokument scheint keine genaue Angabe über die Ausbildung der Ankermittel zu entnehmen zu sein. Gemäß den Figuren scheinen diese jedoch aus sich in Längsrichtung erstreckenden Streben ausgebildet zu sein. In etwa in Querrichtung verlaufende Streben sind diesem Dokument nicht zu entnehmen.

Aus der US 2013/312763 A1 gehen ein Verfahren und eine Vorrichtung zum Verschluss eines Körperlumens, insbesondere eines Eileiters hervor. Gemäß einem Ausführungsbeispiel ist vorgesehen, dass die Vorrichtung aus einer gitterartigen Struktur ausgebildet ist, wobei diese Struktur über einem stangenartigen Basismittel derart angeordnet ist, dass ein Ende der Struktur fest mit diesem Basismittel verbunden ist und ein anderes Ende der Struktur auf dem Basismittel in Längsrichtung derart verschiebbar ist, dass beim Verschieben die Struktur zwei in etwa spindelförmige gitterartige Strukturen ausbildet. Ein zwischen diesen beiden Strukturen ausgebildeter Bereich kann als Verbindungsbereich angesehen werden.

In der US 2012/067352 A1 gehen eine Vorrichtung, ein System und ein Verfahren zum Durchführen von gynäkologischen Prozeduren hervor. In einem Ausführungsbeispiel ist eine gitterartige Struktur gezeigt. Diese gitterartige Struktur ist ein Stent. Dieser Stent weist am distalen und am proximalen Ende einen sich konisch aufweitenden bzw. einen sich konisch verjüngenden Abschnitt auf. Der Stent ist einstückig ausgebildet

Aus der US 2012/192872 A1 geht eine wieder entfernbare rohrförmige Eileitervorrichtung hervor. Die Eileitervorrichtung kann z. B. eine wendelförmige Rahmenstruktur umfassen, wobei diese mit einem flussreduzierenden Mittel, wie z. B. einem folienartigen Überzug bzw. einem dünnen polymerischen Film überzogen ist. Dabei kann ein proximaler Abschnitt der Vorrichtung konisch verjüngend und ein distaler Abschnitt konisch aufweitend ausgebildet sein. Diese Struktur ist einstückig ausgebildet.

Aufgabe der vorliegenden Erfindung ist es, eine Uterotubar-Implantat-Vorrichtung bereitzustellen, die einen zuverlässigen Verschluss des Eileiters zur Empfängnisverhütung ermöglicht und die auf einfache Weise in den menschlichen Körper einbringbar und an den Implantationsort anpassbar, an diesem fixierbar ist und auch wieder aus dem Körper entfernbar ist.

Diese Aufgabe wird durch den unabhängigen Anspruch gelöst. Vorteilhafte Ausgestaltungen sind in den Unteransprüchen angegeben.

Erfindungsgemäß ist eine Uterotubar-Implantat-Vorrichtung vorgesehen. Diese Uterotubar-Implantat-Vorrichtung umfasst zumindest drei komprimierbare selbstexpandierbare Körper, wobei die Körper aus einer Gitterstruktur ausgebildet sind und in einer Längsrichtung miteinander gekoppelt sind, wobei ein Körper der in Längsrichtung distal angeordnet ist und ein Körper der in Längsrichtung proximal angeordnet ist, derart trichterförmig ausgebildet sind, dass der proximale trichterförmige Körper in Längsrichtung von distal nach proximal und der distale Körper von proximal nach distal konisch aufweitend ausgebildet ist, und ein zwischen dem distalen trichterförmigen Körper und dem proximalen trichterförmigen Körper angeordneter Körper derart spindelförmig ausgebildet ist, dass der spindelförmige Körper in Längsrichtung an seinem proximalen Ende derart konisch aufweitend und an seinem distalen Ende derart konisch verjüngend ausgebildet ist, dass der Körper eine Spindelform ausbildet.

Dadurch, dass die Vorrichtung zumindest einen distalen und einen proximalen Körper umfasst, wobei zumindest einer der Körper spindel- oder trichterförmig ausgebildet ist, lässt sich die Vorrichtung optimal an die Biegung und den Durchmesser eines Eileiters anpassen. Das bedeutet, die Vorrichtung ist auf einfache Art und Weise optimal an eine Hohlorganwandung, insbesondere eine Eileiterwandung, anpassbar und stellt eine optimale Passform bereit. Aufgrund der optimalen Passform ist auch eine gute Verankerung mit dem an die Vorrichtung angrenzenden Gewebe des Eileiters möglich.

Unter einer Kopplung der Körper wird im Rahmen der vorliegenden Erfindung verstanden, dass die Körper der erfindungsgemäßen Vorrichtung sowohl vor dem Einbringen als auch nach dem Einbringen miteinander verbunden sind. Die Verbindung kann vorzugsweise entlang einer zentralen Längsachse der Körper erfolgen.

Unter einer Gitterstruktur der Körper kann im Rahmen der vorliegenden Erfindung eine in etwa gleichmäßige Struktur verstanden werden, die sich kreuzende Gitterstreben umfasst, die im sich im Bereich von Kreuzungspunkten überlappen oder miteinander verbunden sind.

Die Körper können im expandierten Zustand eine vorbestimmte radiale Expansionskraft aufweisen. Diese radiale Expansionskraft ist groß genug, den Eileiterabschnitt geringfügig aufzudehnen. Ein Bereich der Körper, der dann im expandierten Zustand am entsprechenden Eileiterabschnitt anliegt, wird als Kontaktabschnitt bezeichnet und ist auf einer Mantelwandung der Körper zur Eileiterwandung hin ausgebildet. Der Kontaktabschnitt kontaktiert derart mit der Eileiterwandung, dass er einen mechanischen Stimulus derart erzeugt, dass zumindest ein Bereich des Kontaktabschnittes der Körper mit der Eileiterwandung verwächst. Gleichzeitig kontaktiert die entsprechende Gitterstruktur derart gleichmäßig und atraumatisch den Eileiterabschnitt, dass die Eileiterwandung bis auf den Wachstumsstimulus nicht gereizt wird und insbesondere eine Verletzung bzw. eine Perforation der Eileiterwandung vermieden wird.

Die zumindest zwei bis vier und vorzugsweise drei Körper können auch unterschiedliche radiale Expansionskräfte aufweisen, um sich an die Krümmung des Eileiters anzupassen.

Der distale und der proximale Körper können vorzugsweise einen größeren Durchmesser und eine größere radiale Expansionskraft als der bzw. die mittleren Körper aufweisen. Der oder die mittleren Körper liegen in der ausgeprägtesten Engstelle des Eileiters (intramuraler Abschnitt), der distale und der proximale Körper sind im Eileiter dahinter bzw. davor angeordnet. Auf diese Weise wird eine Dislokation des Implantates verhindert. Eine Dislokation (in beide Richtungen) wäre nur dann möglich, wenn einer der äußeren Körper mit großer radialer Expansionskraft in der Lage wäre, den intramuralen Abschnitt zu durchwandern. Dies wird durch den größeren Durchmesser und die größeren radialen Expansionskräfte des distalen und des proximalen Körpers gegenüber dem oder den mittleren Körpern verhindert.

Die Körper können vorzugsweise rotationssymmetrisch ausgebildet sein.

Das erfindungsgemäße Implantat weist aufgrund der Anordnung und der Ausbildung seiner tubulären, vorzugsweise rotationssymmetrischen Wandungen eine gute Anpassungsfähigkeit an die individuelle uterotubare Anatomie auf. Zum einen ermöglichen die zumindest zwei Körper eine feste Verankerung im Eileiter und zum anderen sind sie derart nachgiebig bzw. flexibel, dass keine chronischen Schmerzen und Entzündungsreaktionen auftreten.

Die Anpassungsfähigkeit an den Eileiter, der ein Hohlorgan mit interindividuell (von Patientin zu Patientin) unterschiedlicher Krümmung und wechselndem Durchmesser ist, ergibt sich durch die axiale Kopplung und Anordnung der einzelnen Körper, sowie durch die Mantelwandungen der Gitterstrukturen der Körper, die aufgrund ihrer radialen Expansionskraft eine möglichst große Kontaktfläche mit der Eileiter-Innenwandung, bei gleichmäßigem Expansionsdruck bildet (punktuell hoher Druck auf die Eileiterwand fördert die Perforation). Eine selbstexpandierbare tubuläre Gitterstruktur ist vorteilhaft, weil sie bezüglich der Federwirkung ihrer radialen Expansion sehr fein eingestellt werden kann, so dass sie optimal auf die Compliance (im physiologischen Sinne: ΔV/Δp) des Hohlorgans (Eileiter) abgestimmt werden kann. Ein Optimum ist erreicht, wenn das Implantat nicht zu fest sitzt, da sonst die Gefahr von Trauma, Nekrose, und Perforation bestünde, aber auch nicht zu locker sitzt, da das Implantat sonst aus dem Eileiter herausrutschen oder diesen nicht richtig verschließen würde.

Das Implantat kann hysteroskopisch oder durchleuchtungsgesteuert über einen dünnen Katheter im komprimierten Zustand in den Uterotubarwinkel und den proximalen Eileiter eingeführt und dort entfaltet werden. Nach Entfaltung verbleibt das Implantat ortsstabil im proximalen Eileiter und im Uterotubarwinkel und wächst in die Uterotubarschleimhaut ein. Die Verschlusswirkung ist damit zunächst mechanisch. Durch eine zusätzliche medikamentöse Dotierung des Implantats oder von dessen Teilkomponenten kann eine zusätzliche pharmakologische Wirkung erzielt werden, solange das Medikament wirksam ist.

Bei der erfindungsgemäßen Vorrichtung ist es, im Gegensatz zur der in der US 2015/0201945 A1 offenbarten Vorrichtung, vorteilhaft, dass sich die Körper der Vorrichtung auf Grund Ihrer selbstexpandierbaren Gitterstruktur, Ihrer Form und Ihrer geringen axialen Steifigkeit sowie Ihrer dosierten radialen Expansionskraft und Compliance an eine Wandung des Lumens des Eileiters mit größtmöglicher Kontaktfläche anschmiegen und daher beim Patienten nach der Implantation in der Regel keinerlei Beschwerden verursachen. Somit kommt es bei Verwendung der erfindungsgemäßen Vorrichtung zu keiner unphysiologischen axialen Streckung des gekrümmten Eileiters und zu keiner punktuell hohen Belastung der Eileiterwand, die zu einem Trauma, einer Perforation oder Nekrose führen könnte. Damit bietet die erfindungsgemäße Vorrichtung einen hohen "Tragekomfort" bzw. eine beschwerdefreie Benutzung.

Die Körper können im expandierten Zustand unterschiedliche Durchmesser aufweisen, wobei der distale Körper im expandierten Zustand einen geringeren Durchmesser aufweisen kann als der proximale Körper. Der proximale und der distale Körper können im expandierten Zustand vorzugsweise einen größeren Durchmesser aufweisen als der mittlere bzw. die mittleren Körper. Der Durchmesser eines Körpers wird in dem Bereich betrachtet, an dem die Mantelwandung des Körpers den größten Durchmesser im expandierten Zustand aufweist.

Der in der Uteruswand verlaufende intramurale Teil des Eileiters hat den kleinsten Durchmesser. Während er zum Uteruskavum hin eine trichterförmige Öffnung (Ostium uterinum) aufweist, kann der Durchmesser innerhalb der Uteruswand weniger als 1 mm betragen. Daran schließt sich der sogenannte isthmische Teil des Eileiters an. Er ist 2 bis 3 cm lang, hat einen Durchmesser von 2 bis 3 mm mit einer Tendenz zu einem größeren Durchmesser weiter distal, wo sich der ampulläre Abschnitt mit einem Durchmesser von 4 bis 10 mm anschließt. Der isthmische Teil besitzt eine ausgeprägte Wandmuskulatur, die aufgrund ihrer Kontraktion auf ein dort befindliches Implantat einwirken kann, mit der Gefahr, dieses durch peristaltische Aktivität zu entfernen.

Die genannten Eileiterabschnitte werden mit der erfindungsgemäßen Vorrichtung verschlossen. Der für das Implantat vorgesehene Eileiterabschnitt ist somit ein Hohlorgan mit einem sehr engen Segment. Proximal davon ist das Uteruskavum, distal der relativ weite ampulläre Teil des Eileiters. Daher ermöglicht die Ausbildung der Vorrichtung eine optimale Anpassbarkeit an die Form bzw. Geometrie des Eileiterkanals in diesem Abschnitt.

Gemäß dem bevorzugten Ausführungsbeispiel umfasst die Vorrichtung in Längsrichtung von distal nach proximal einen ersten Körper mit größeren Durchmesser, der zur Implantation im isthmischen Abschnitt vorgesehen sind, einen zweiten Körper mit kleinerem Durchmesser zur Implantation im intramuralen Abschnitt, und einen proximalen Körper mit größerem Durchmesser zur Implantation im Uteruscavum. Der proximale Körper kann wiederum einen größeren Durchmesser aufweisen als der distale Körper.

Wenn das Implantat lediglich aus zwei Körpern ausgebildet ist, sind diese derart angeordnet, dass die ausgeprägteste Engstelle des Eileiters zwischen diesen beiden Körpern angeordnet ist. Auf diese Weise wird das Implantat nicht durch die Motilität der Tubenmuskulatur disloziert (d.h. weder in das Uteruskavum ausgestoßen, noch in den ampullären Teil verlagert). D.h. der proximale Körper ist so groß bzw. derart bemessen, dass ein komplettes Einziehen des Implantats in den Eileiter verhindert wird, und der distale Körper ist so groß bzw. derart bemessen, dass ein komplettes Ausstoßen des Implantats in das Uteruskavum verhindert wird. Wenn die Vorrichtung aus drei Körpern ausgebildet ist können entsprechend, wie vorstehend beschrieben, der distale und der proximale Körper einen größeren Durchmesser als der oder die mittleren Körper aufweisen, um ein Dislokation zu verhindern.

Einer der Körper kann spindelförmig und der andere Körper kann trichterförmig ausgebildet sein.

Gemäß diesem bevorzugten Ausführungsbeispiel umfasst die Vorrichtung drei Körper, wobei alle drei Körper spindelförmig ausgebildet sind. Alternativ können auch der mittlere und der distale Körper spindelförmig und der proximale Körper trichterförmig ausgebildet sein. Alternativ können auch der proximale und der mittlere Körper spindelförmig und der distale Körper trichterförmig ausgebildet sein. Auch die Spindelform ist günstig um eine Dislokation zu verhindern, da sie eine annähernd zylindrische Mantelfläche bereitstellt, die sich relativ gleichmäßig mit einer gut dosierten Federkraft (Compliance) an die Eileiterwand anlegt.

Alternativ können auch der proximale und der distale Körper trichterförmig und der mittlere Körper spindelförmig ausgebildet sein. Der proximale Körper kann dann von distal nach proximal oder von proximal nach distal konisch aufweitend ausgebildet sein Der distale Körper kann dann von distal nach proximal oder von proximal nach distal konisch aufweitend ausgebildet sein. Bevorzugt ist jedoch, dass gemäß einer solchen Ausbildung der Vorrichtung der proximale Körper von distal nach proximal und der distale Körper von proximal nach distal konisch aufweitend ausgebildet sind. Dies verhindert äußerst effektiv eine Dislokation der Vorrichtung im Eileiter. Die Trichterform hingegen sperrt gegen eine Dislokation weil sich das Implantat damit in der Eileiterwand verkeilt. Die Eigenbewegung des Eileiters zum "Ausmelken" natürlicherweise eines Eies (aber eben auch des Implantats) kann in beide Richtungen gehen, insbesondere aber in Richtung des Uteruscavums. Ideal kann somit eine Ausbildung der Vorrichutng mit zwei solchen "Sperrtrichtern" an beiden Enden sein.

Eine weitere effektive Möglichkeit, um eine Dislokation zu verhindern besteht darin, wie vorstehend aufgezeigt, die größeren Durchmesser und die größeren radiale Expansionskräfte an den äußeren spindelförmigen Körper vorzusehen, die derart abgeordnet sind, dass sich der enge intramuraler Abschnitt genau zwischen diesen beiden befindetbefindet.

Unter einem spindelförmigen Körper wird im Rahmen der vorliegenden Erfindung ein Körper verstanden, der bzgl. der Längsrichtung in seiner Mitte den größten Durchmesser aufweist und der an seinem proximalen Ende derart konisch aufweitend und an seinem distalen Ende derart konisch verjüngend ausgebildet ist, dass der Körper eine Spindelform ausbildet, bzw. in etwa elliptisch im Längsschnitt ausgebildet ist. Das bedeutet, der Körper weist an seinem distalen und an seinem proximalen Ende den geringsten Durchmesser auf. Das heißt, das distale und das proximalen Ende des spindelförmigen Körpers sind in etwa strangförmig, wobei dieser durch Zusammenfassung (d.h., dauerhafte axiale Kompression, zum Beispiel durch Aufziehen einer Hülse, durch Löten, Schweißung oder Klebung) der Gitterstruktur ausgebildet ist. Eine solche Zusammenfassung begrenzt die Körper bzw. bildet den Übergang von einem Körper zum nächsten Körper aus.

Der Bereich eines solchen Körpers, der zwischen dem proximalen Ende und an dem distalen Ende angeordnet ist kann auch in etwa zylindrisch ausgebildet sein.

Ein trichterförmiger Körper ist ein Körper, der in Längsrichtung von distal nach proximal oder von proximal nach distal über seine ganze bzw. über seine im Wesentlichen ganze Länge konisch aufweitend ausgebildet ist. Eine umgekehrte Anordnung ist ebenfalls möglich. Unter einer Trichterform werden auch glockenförmige oder konische Formen mit unterschiedlichen Neigungswinkeln bezüglich der Längsrichtung verstanden.

Weiterhin können ein flexibler Verbindungsdraht und/oder Verbindungsdrahtabschnitte vorgesehen sein, über den bzw. die die Körper miteinander gekoppelt sind.

Der Verbindungsdraht bzw. die Verbindungsdrahtabschnitte dienen ähnlich wie der Bereich bzw. die Bereiche, in denen die Körper miteinander gekoppelt sind als Abstandshalter, der den Abstand der einzelnen Körper in Längsrichtung zueinander definiert. Zudem sind die Körper über den Verbindungsdraht bzw. die Verbindungsdrahtabschnitte miteinander gekoppelt. Der Verbindungsdraht bzw. die Verbindungsdrahtabschnitte nehmen Druck- und Zugkräfte auf, um die Anordnung der Körper zueinander zu erhalten und eine Dislokation des Implantats zu verhindern, wobei der Draht selbst flexibel ausgebildet sein sollte, um eine Anpassung an Krümmungen des Eileiters zu ermöglichen.

Der Verbindungsdraht bzw. die Verbindungsdrahtabschnitte können sich durch die Körper in Längsrichtung hindurch erstrecken, wobei dann die Körper zumindest an ihren proximalen und/oder distalen Enden mit dem Verbindungsdraht derart verbunden sind, dass die Kopplung der Körper mittels des Verbindungsdrahtes erfolgt.

Weiterhin können die Körper über Verbindungsdrahtabschnitte perlenkettenartig miteinander gekoppelt sein. Das bedeutet, ein distales bzw. ein proximales Ende eines Körpers ist mit einem proximalen bzw. einem distalen Ende eines weiteren Körpers über einen Verbindungsdrahtabschnitt gekoppelt. Der Verbindungsdraht bzw. die Verbindungsdrahtabschnitte sind in radialer Richtung flexibel und weisen in Längsrichtung eine gewisse Steifigkeit gegenüber Druck und Zug auf.

Ein proximales Ende eines spindelförmigen Körpers kann beispielsweise mittels einer rohrförmigen Hülse mit dem Verbindungsdraht derart gekoppelt sein, dass das proximale Ende dieses Körpers in Längsrichtung verschiebbar mit dem Verbindungsdraht verbunden ist. Ein distales Ende eines spindelförmigen Körpers kann beispielsweise mittels einer rohrförmigen Hülse mit dem Verbindungsdraht fest verbunden sein. Eine umgekehrte Anordnung ist ebenfalls möglich.

Somit können ein distales und/oder ein proximales Ende eines spindelförmigen Körpers oder ein zusammengeführtes Ende eines trichterförmigen Körpers mit einem Verbindungsdrahtabschnitt gekoppelt sein, um diesen Körper mit einem weiteren Körper des Implantats zu verbinden.

Durch die Verschiebbarkeit eines vorzugsweise proximalen Endes oder auch des distalen Endes des spindelförmigen Körpers bezüglich des Verbindungsdrahtes in Längsrichtung ist es möglich, dass der entsprechende Körper von einem komprimierten in einen radial expandierten Zustand und umgekehrt übergehen kann. Dies ist insbesondere beim Einbringen der erfindungsgemäßen Vorrichtung in den Eileiter mittels eines Katheters wichtig, da die Körper im komprimierten Zustand eine wesentlich größere Erstreckung in Längsrichtung bzw. eine größere Länge aufweisen als im expandierten Zustand, bei dem sich die Länge aufgrund der Expansion in radialer Richtung vermindert.

Die erfindungsgemäße Vorrichtung umfasst zumindest drei oder vier oder fünf oder sechs oder mehr und vorzugsweise drei selbstexpandierbare Körper.

Dabei können Körper spindelförmig ausgebildet sein. Es ist auch möglich, dass Körper trichterförmig ausgebildet sind. Auch Kombinationen aus spindelförmigen und trichterförmigen Körpern sind vorteilhaft. Die bevorzugten Ausführungsformen der Körper werden nachfolgend in den Ausführungsbeispielen noch detaillierter beschrieben.

Die Länge der Vorrichtung vom distalen Ende des distalen Körpers bis zum proximalen Ende des proximalen Körpers beträgt in etwa 10 mm bis 70 mm bzw. 20 mm bis 60 mm bzw. 35 mm bis 45 mm.

Somit beträgt die Länge des Implantats vorzugsweise weniger als 50% bezogen auf eine Gesamtlänge eines Eileiters von in etwa 10 cm bis 16 cm.

Die Körper weisen im expandierten Zustand in den Bereichen Ihrer größten Erstreckung quer zur Längsrichtung der Vorrichtung einen Durchmesser von in etwa 1 bis 3 mm auf.

Die Körper können im expandierten Zustand unterschiedliche Durchmesser aufweisen, wobei ein Körper, der im Uteruskavum bzw. Uterotubarwinkel anordbar ist, als distaler Körper bezeichnet wird, der einen Durchmesser von in etwa 3 mm bis 4 mm und vorzugsweise von 3 mm aufweist und/oder ein oder zwei Körper zur Implantation im intramuralen Abschnitt vorgesehen sind und der oder die Körper als mittlere Körper bezeichnet werden, wobei der oder die Körper einen Durchmesser von in etwa 2 mm aufweisen und ein Körper zur Implantation im isthmischen Abschnitt vorgesehen ist, wobei dieser Körper als proximaler Körper bezeichnet wird, und der distale Körper einen Durchmesser von in etwa 3 bis 4 mm und vorzugsweise von 4 mm aufweist.

Die Körper können aus einem oder mehreren Drähten geflochten oder aus einem rohrförmigen Körper mittels Laserschneiden hergestellt sein.

Als Flecht-Draht ist vorzugsweise ein Nitinoldraht (Form-Gedächtnis-Legierung) vorgesehen. Durch die Formgebung bei der Temperung der Form-Gedächtnis-Legierung kann die radiale Expansionskraft beeinflusst bzw. eingestellt werden.

Die einzelnen Körper können ausgebildet werden, in dem ein fortlaufendes Geflecht, welches eine Basisstruktur der Vorrichtung darstellt, punktuell komprimiert wird, bspw. durch Aufziehen einer Hülse, durch Laserschweißen oder Löten oder Kleben.

Die Vorrichtung bzw. deren Körper können alternativ aus einem Rohr, zum Beispiel aus Nitinol (Formgedächtnislegierung Nickel Titan), mittels Laserschneiden hergestellt werden. Anschließend wird die zu erzeugende Form im expandierten Zustand auf das Rohr aufgeprägt. Die Verbindungspunkte zwischen den Körpern entstehen entweder sekundär durch punktuelle Kompression des expandierten Rohres (fixiert durch Aufziehen einer Hülse, durch Laserschweißen, durch Löten oder Kleben) oder primär durch Belassung als nicht durch Laserschnitt bearbeiteter kurzer Rohrabschnitt mit kleinem Ausgangsdurchmesser.

Beim Laserschneiden kann die radiale Expansionskraft durch die folgenden Parameter eingestellt werden
- durch das Schnittmuster des Laserschnitt-Gitters, und
- durch den Durchmesser, die Dicke und das Material des Rohr-Halbzeugs und dessen Formgebung bei Temperung, wobei vorzugsweise eine Formgedächtnis-Legierung wie z.B. Nitinol eingesetzt wird.

Auch eine solche Laser-geschnittene Vorrichtung ist im komprimierten Zustand im Implantationskatheter angeordnet und expandiert selbständig bzw. ist selbstexpandieren nach dem der Katheter entfernt wurde bzw. nach dem die Vorrichtung freigesetzt wurde.

Die Öffnungen der Gitterstruktur bzw. des geschnittenen Körpers weisen bevorzugt eine Öffnungsweite bzw. einen Strebenabstand von kleiner 1 mm bzw. von 200 µm bis 700 µm, insbesondere von 300 µm bis 600 auf. Tendenziell sind bei einer Herstellung durch Flechten von Einzeldrähten die durch die Gitterstruktur bzw. Streben gebildeten Rauten in axialer Richtung länger, wenn ein Flechtwinkel geringer 45 °, bzw. kürzer, wenn ein Flechtwinkel größer 45° verwendet wurde.

Die Mantelflächen eines oder mehrerer Körper können ganz oder zumindest teilweise beschichtet sein. Die Art und der Aufbau der Beschichtung werden nachfolgend noch detaillierter beschrieben.

Die Beschichtung kann bspw. ein quellbares Hydrogel sein, welches mit einem bioaktiven Wirkstoff dotiert ist. Der Wirkstoff kann beispielsweise ein Spermizid, wie z. B. Nonoxinol-9 sein. Weiterhin kann ein derartiger Wirkstoff ein Anti-Progesteron sein, wodurch die Blutungsneigung reduziert und das Einwachsen des Implantats in die Eileiterwandung gefördert bzw. unterstützt wird

Weiterhin können die spindelförmigen Körper mit einem verschlussfördernden Material gefüllt sein, das ebenfalls mit einem bioaktiven Wirkstoff dotiert sein kann. Die Art und der Aufbau des verschlussfördernden Materials werden nachfolgend noch detaillierter beschrieben.

Im Falle, dass als verschlussförderndes Material quellbare Fasern oder dergleichen vorgesehen sind, führt dessen Quelleigenschaft beim Kontakt mit Flüssigkeit zu einer Verstärkung der Verschlusswirkung der Vorrichtung.

Beim Vorsehen eines Hydrogels oder dergleichen als verschlussförderndes Material, welches beispielsweise mit einem bioaktiven Wirkstoff dotiert ist, kann diese Dotierung zur Reduzierung der Spermienbeweglichkeit beitragen, so dass die kontrazeptive Wirkung verstärkt wird.

Weiterhin können Fasern in die Gitterstruktur eingewoben sein.

Die Fasern können zur Erhöhung der spermiziden Wirkung vorgesehen und aus Kupfer oder auch zur Erhöhung der Verschlusswirkung aus quellbaren Polymerfasern ausgebildet sein.

Unter einem Einweben wird im Rahmen der vorliegenden Erfindung auch ein Flechten oder ein andersartiges Einbringen der Fasern zwischen die Gitterstruktur verstanden.

Am distalen Ende des distalen Implantats kann eine Sondenspitze vorgesehen sein, die beispielsweise als eine atraumatische Kugel ausgebildet ist. Eine derartige atraumatische Kugel erleichtert das Einbringen der Vorrichtung zum Implantationsort und verhindert eine Verletzung des beim Einfügen die Vorrichtung umgebenden Gewebes. Eine solche atraumatische Kugel kann mit dem distalen Ende des Implantats über einen geraden oder gekrümmten Drahtabschnitt verbunden sein.

Grundsätzlich können die weiteren Körper des Implantats bis auf einen spindel- oder trichterförmig ausgebildeten Körper auch andere Formen aufweisen, die zur Anpassung an die Form des Eileiters geeignet sind, bspw. in etwa kugelförmig oder zylindrisch.

Die Erfindung wird im Folgenden anhand der Zeichnungen näher erläutert. In den Figuren 1 bis 12 sind Uterotubar-Vorrichtungen dargestellt, die das Verständnis der vorliegenden Erfindung erleichtern. Diese zeigen in
Fig. 1 eine Uterotubar-Implantat-Vorrichtung in einem am Implantationsort bzw. in einem Eileiter eingebrachten Zustand,
Fig. 2 eine Vorrichtung in einer schematischen seitlichen Ansicht,
Fig. 3 eine weitere Vorrichtung in einer schematischen seitlichen Ansicht,
Fig. 4 eine weitere Vorrichtung in einer schematischen seitlichen Ansicht,
Fig. 5 eine weitere Vorrichtung in einer schematischen seitlichen Ansicht,
Fig. 6 eine weitere Vorrichtung in einer schematischen seitlichen Ansicht,
Fig. 7 eine Detailansicht eines Endes eines trichterförmigen Körpers der erfindungsgemäßen Vorrichtung,
Fig. 8 eine Uterotubar-Implantat-Vorrichtung in einem am Implantationsort bzw. in einem Eileiter eingebrachten Zustand,
Fig. 9 eine geflochtene Vorrichtung in einer schematischen seitlichen Ansicht,
Fig. 10 eine lasergeschnittenen Vorrichtung in einer schematischen seitlichen Ansicht,
Fig. 11 eine Vorrichtung in einer schematischen seitlichen Ansicht,
Fig. 12 eine Vorrichtung in einer schematischen seitlichen Ansicht, und
Fig. 13 eine bevorzugte Ausführungsform einer erfindungsgemäßen Vorrichtung in einer schematischen seitlichen Ansicht.

Eine erfindungsgemäße Uterotubar-Implantat-Vorrichtung 1 umfasst zumindest zwei komprimierbare selbstexpandierbare Körper 2, wobei die Körper aus einer Gitterstruktur 3 ausgebildet sind. Die Körper 2 sind in einer Längsrichtung 4 miteinander derart gekoppelt, dass einer der Körper 2 in Längsrichtung distal 5 und der andere Körper 2 in Längsrichtung 4 proximal 6 angeordnet ist.

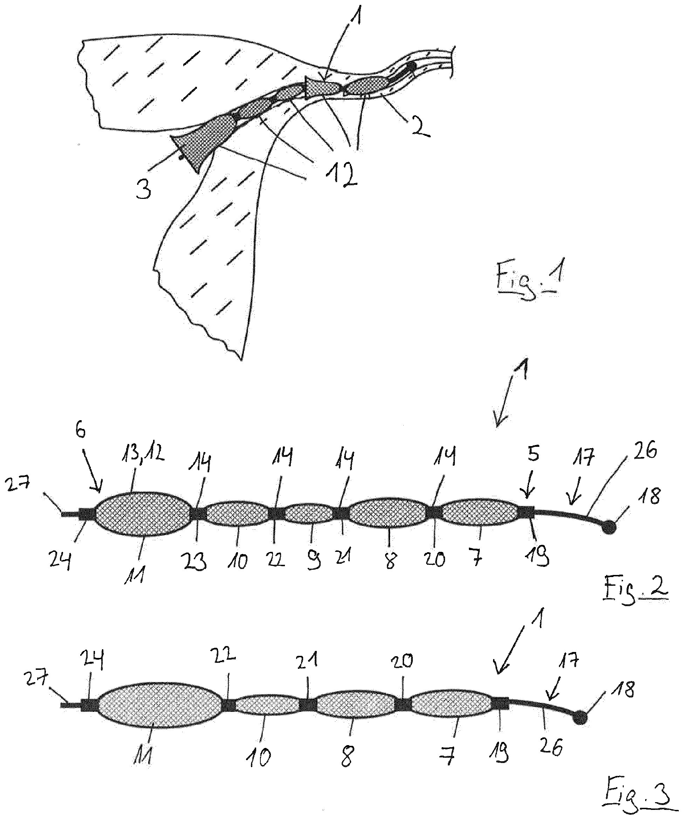

Im Folgenden wird eine Uterotubar-Implantat-Vorrichtung 1 anhand eines ersten Ausführungsbeispiels näher beschrieben (Figuren 1 und 2).

Gemäß diesem ersten Ausführungsbeispiel umfasst die Vorrichtung 1 in Längsrichtung 4 von distal 5 nach proximal 6 einen ersten und einen zweiten Körper 7, 8, die zur Implantation im isthmischen Abschnitt vorgesehen sind, einen dritten und einen vierten Körper 9, 10 zur Implantation im intramuralen Abschnitt, und einen proximalen Körper 11 zur Implantation im Uteruscavum.

Die Körper 2, 7, 8, 9, 10 und 11 sind komprimier- und selbst expandierbar ausgebildet. Auf diese Weise ist es möglich, die Vorrichtung 1 minimal-invasiv mittels eines dünnen Katheters (nicht dargestellt) zu implantieren. Der Katheter kann hysteroskopisch oder unter Durchleuchtungskontrolle zum Implantationsort bewegt werden.

Die Körper 2, 7, 8, 9, 10 und 11 weisen im expandierten Zustand eine vorbestimmte radiale Expansionskraft auf. Diese Expansionskraft ist groß genug bzw. derart bemessen, dass die Körper einen Eileiterabschnitt, den sie kontaktieren, geringfügig aufdehnen können, um einen vollumfänglichen Kontakt der Körper zur Eileiterwandung zu bewirken. Dieser Kontakt wirkt als mechanischer Stimulus für ein Einwachsen der Körper in die Eileiterwand. Zudem muss die radiale Expansionskraft derart bemessen sein, dass ein Kontaktabschnitt 12 (Figur 1, 2) einer Mantelwandung der Körper 13 die Eileiterwandung derart kontaktiert, dass keine als schmerzhaft empfundene chronische Reizung oder gar Penetration der Eileiterwandung auftritt. Das bedeutet, die Expansionskraft muss derart bemessen sein, dass der Kontaktabschnitt 12 der Mantelwandung 13 der Körper atraumatisch mit dem entsprechenden Abschnitt der Eileiterwandung kontaktiert.

Die Körper 2, 7, 8, 9, 10 und 11 sind aus mehreren Drähten derart geflochten, dass eine geflochtene tubuläre Endlos-Struktur entsteht. Diese Struktur ist entsprechend einer Gesamtlänge der Vorrichtung 1 von in etwa 35 mm an dem proximalen und dem distalen Ende 5, 6 abgelängt.

Am proximalen und am distalen Ende 6, 5 sowie an vier dazwischen liegenden Kopplungsabschnitten 14 sind die Körper eingeschnürt bzw. komprimiert. Die Komprimierung kann beispielsweise mittels Schweißpunkten, Kleben oder einer aufgezogenen Hülse erfolgen.

Gemäß dem ersten Ausführungsbeispiel sind Hülsen 19, 20, 21, 22, 23, 24 vorgesehen. Die Bereiche in denen die Hülsen 20, 21, 22, 23 angeordnet sind bilden Kopplungsabschnitte 14 aus.

Als Flecht-Draht ist vorzugsweise ein Nitinoldraht (Form-Gedächtnis-Legierung) vorgesehen. Durch die Formgebung bei der Temperung der Form-Gedächtnis-Legierung kann die radiale Expansionskraft beeinflusst bzw. eingestellt werden. Weiterhin lässt sich die radiale Expansionskraft durch die Art der Gitterstruktur 3 bzw. des Drahtgeflechts der Körper, die Anzahl der Drähte, den Durchmesser der Drähte und der Körper und den Flechtwinkel der Drähte beeinflussen. Es ist ein Flechtwinkel von vorzugsweise größer 35° bezogen auf eine axiale Mittelachse vorgesehen, um eine ausreichende Selbstexpandierbarkeit zu erzielen.

Die in der Gitterstruktur 3 der Körper ausgebildeten Gitteröffnungen weisen vorzugsweise eine maximale Weite bzw. einen Strebenabstand von kleiner 1 mm bzw. von 200 µm bis 700 µm, insbesondere von 300 µm bis 600 auf.

Am distalen Ende 5 des Implantats 1 ist eine Sondenspitze 17 angeordnet. Die Sondenspitze 17 umfasst vorzugsweise einen Drahtabschnitt 26 und eine daran angeordnete atraumatische Kugel 18.

Die Streben bzw. geflochtenen Drähte, die die Mantelflächen der Körper bilden, sind vorzugsweise ganz oder zumindest teilweise beschichtet. Die Beschichtung (nicht dargestellt) kann ein Polymer umfassen, wie z. B. Polyurethan oder ein bioabbaubares Polylactid oder auch ein quellbares Hydrogel. Die Beschichtung kann prinzipiell aus allen Kunststoffen ausgebildet sein, in die sich Medikamente einlagern lassen und die eine Wirkstoffabgabe über einen längeren Zeitraum ermöglichen.

Weiterhin kann die Beschichtung, insbesondere wenn ein quellbares Hydrogel vorgesehen ist, mit einem bioaktiven Wirkstoff dotiert sein. Der Wirkstoff kann beispielsweise ein Spermizid, wie z. B. Nonoxinol-9 sein. Weiterhin kann ein derartiger Wirkstoff ein Anti-Progesteron sein, wodurch die Blutungsneigung reduziert und das Einwachsen des Implantats in die Eileiterwandung gefördert bzw. unterstützt wird.

Die spindelförmigen Körper 2, 7, 8, 9, 10 und 11 gemäß dem ersten Ausführungsbeispiel sind zusätzlich oder alternativ zur Beschichtung mit einem verschlussfördernden Material (nicht dargestellt) gefüllt. Als verschlussförderndes Material sind bspw. biokompatible Textilfasern aus Polyethylentereftalat, Polyamid oder bioabbaubaren Polylactid vorgesehen. Durch eine Quellfähigkeit derartiger Fasern beim Kontakt mit Flüssigkeit wird die Verschlusswirkung gefördert. Die Fasern können auch mit Hydrogel imprägniert sein, um die Quellfähigkeit zu erhöhen.

Zusätzlich und/oder alternativ sind die Gitterstrukturen der Körper direkt mit quellbarem Hydrogel beschichtet. Sowohl die Fasern und/oder das Hydrogel können mit einem bioaktiven Wirkstoff dotiert sein.

Bei der Biodegradierung von Polylactid entstehen saure Abbauprodukte. In einem derart sauren Milieu ist die Spermienbeweglichkeit erheblich reduziert. Auf diese Weise kann die kontrazeptive Wirkung verstärkt werden.

Zusätzlich und/oder alternativ können zur Erhöhung der spermiziden Wirkungen Fäden in die Gitterstruktur der Implantatkörper mittels Weben oder Flechten eingebracht sein. Hierbei sind insbesondere Kupfer- oder Polylactidfäden vorgesehen.

Zusätzlich und/oder alternativ können zur Erhöhung der Verschlusswirkung quellbare Polymerfasern in die Gitterstruktur eingewoben oder eingeflochten sein. Es ist auch möglich, dass die vorstehend beschriebene Füllung Kupfer enthält, um die spermizide Wirkung zu erhöhen, bzw. Gold oder Silber enthält, um eine bakterizide Wirkung auszuüben.

Die Länge der erfindungsgemäßen Vorrichtung von einem distalen Ende des ersten Körpers 7 bis zu einem proximalen Ende des fünften Körpers 11 beträgt 10 mm bis 70 mm bzw. 20 mm bis 60 mm und vorzugsweise 35 mm bis 45 mm.

Die Körper sind vorzugsweise rotationssymmetrisch ausgebildet.

Weiterhin können die Körper unterschiedliche Längen aufweisen.

Gemäß dem ersten Ausführungsbeispiel weist der im Uteruscavum anordbare fünfte Körper 11 einen Durchmesser von in etwa 3 bis 4 mm auf und der dritte und der vierte Körper 9, 10 weisen im expandierten Zustand einen Durchmesser von in etwa 2 mm auf, die zur Implantation im intramuralen Abschnitt vorgesehen sind und der erste und der zweite Körper 7, 8, die zur Implantation im isthmischen Abschnitt vorgesehen sind, weisen einen Durchmesser von in etwa 3 mm im expandierten Zustand auf. Die Toleranz der expandierten Körper beträgt in etwa +/-1mm.

Gemäß einer alternativen Ausführungsform des ersten Ausführungsbeispiels (nicht dargestellt) kann auch ein Verbindungsdraht 16 vorgesehen, der sich durch die Körper in Längsrichtung 4 hindurcherstreckt.

Gemäß dieser ersten Ausführungsform ist der Verbindungsdraht 16 ein einzelner, durchgehender Draht aus Metall oder Kunststoff. Alternative Ausführungsformen des Verbindungsdrahtes werden nachfolgend noch detaillierter beschrieben.

Im Falle, dass der Verbindungsdraht 16 aus Kunststoff ausgebildet ist, kann dieser beispielsweise aus einem bioabbaubaren Polylactid ausgebildet sein.

Die Körper 2, 7, 8, 9, 10 und 11 sind mit dem Verbindungsdraht lediglich am distalen Ende über eine distale Hülse 19 verbunden. Die distale Hülse 19 ist mit dem Verbindungsdraht 16 fest verbunden. Die übrigen zwischen den Körpern angeordneten Hülsen 20, 21, 22, 23 und 24 sind mit dem Verbindungsdraht derart gekoppelt, dass alle distalen und proximalen Enden der Körper bzw. die entsprechenden Hülsen 20 bis 24 bis auf die fest mit dem Verbindungsdraht verbundene distale Hülse 19 in Längsrichtung verschiebbar mit dem Verbindungsdraht verbunden sind.

Auch der Verbindungsdraht 16 kann Kupfer und/oder Gold und/oder Silber enthalten bzw. mit Kupfer und/oder Gold und/oder Silber beschichtet sein.

Im Folgenden wird ein zweites Ausführungsbeispiel (Figur 3) erläutert.

Sofern nichts anderes beschrieben ist, entspricht dieses Ausführungsbeispiel dem ersten Ausführungsbeispiel und umfasst alle Merkmale des ersten Ausführungsbeispiels. Gleiche Teile sind mit den gleichen Bezugszeichen versehen.

Gemäß dem zweiten Ausführungsbeispiel sind anstelle von fünf spindelförmigen Körpern lediglich vier spindelförmige Körper vorgesehen. Dabei wurde auf den vierten Körper verzichtet, so dass nach dem dritten Körper unmittelbar im Anschluss der fünfte Körper angeordnet ist. Hierbei weist der fünfte Körper eine größere Länge in Längsrichtung auf als im ersten Ausführungsbeispiel.

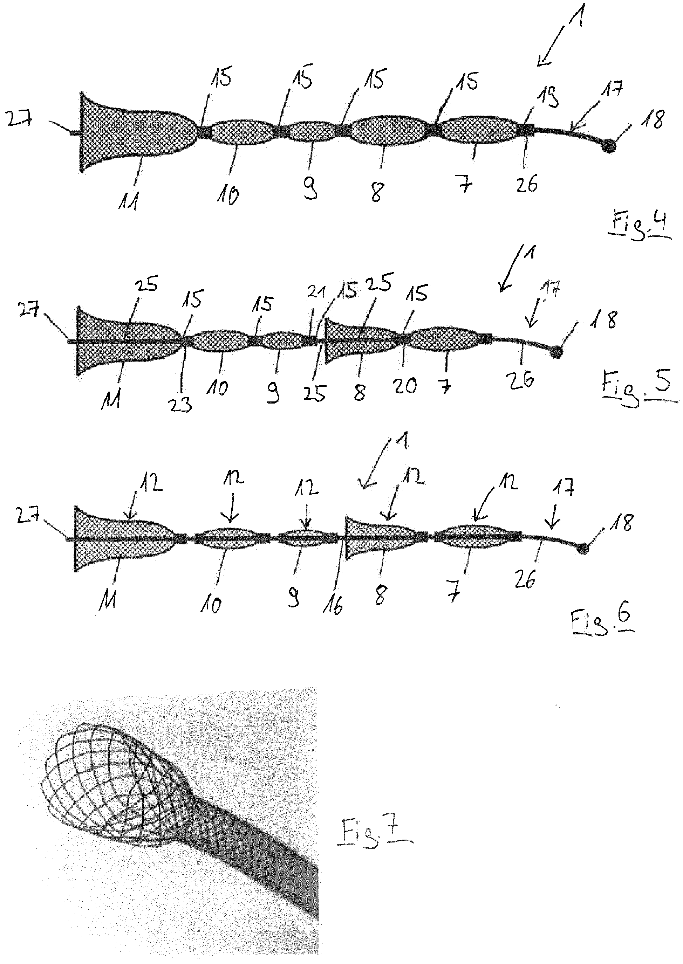

Im Folgenden wird ein drittes Ausführungsbeispiel (Figur 4) der Vorrichtung beschrieben. Sofern nichts anderes beschrieben ist, umfasst das dritte Ausführungsbeispiel dieselben Merkmale wie das erste Ausführungsbeispiel. Gleiche Teile sind mit den gleichen Bezugszeichen versehen.

Gemäß dem dritten Ausführungsbeispiel ist der proximale bzw. der fünfte Körper 11 nicht spindelförmig, sondern trichterförmig, insbesondere glockenförmig ausgebildet.

Hierbei ist vorgesehen, dass ein distales Ende 5 des fünften Körpers 11 mittels einer Hülse mit einem proximalen Ende des vierten Körpers 10 gekoppelt ist.

Im Folgenden wird ein viertes Ausführungsbeispiel beschrieben (Figur 5).

Sofern nichts anderes beschrieben ist, entspricht dieses Ausführungsbeispiel dem dritten Ausführungsbeispiel und weist die gleichen Merkmale auf. Gleiche Teile sind mit den gleichen Bezugszeichen versehen.

Beim vierten Ausführungsbeispiel ist der zweite Körper 8 nicht spindelförmig, sondern glockenförmig ausgebildet. Hierbei ist vorgesehen, dass die distale Hülse 20 die am distalen Ende des ersten glockenförmigen Körpers 8 angeordnet ist, fest mit einem Verbindungsdrahtabschnitt 25 verbunden ist.

Der Verbindungsdrahtabschnitt 25 ist mit der distalen Hülse 21 des spindelförmigen Körpers 9 fest verbunden.

Der zweite glockenförmige Körper 8 ist gemäß diesem Ausführungsbeispiel somit nicht unmittelbar mit dem dritten Körper 9 verbunden. Die Kopplung der beiden distalen Körper 7 und 8 mit den drei proximalen Körpern 9, 10, 11 erfolgt über den Verbindungsdrahtabschnitt 25.

Der dritte und der vierte Körper 9, 10 sind erneut spindelförmig ausgebildet und der fünfte Körper 11 ist wie im dritten Ausführungsbeispiel beschrieben glockenförmig ausgebildet. Hierbei ist vorgesehen, dass der vierte Körper 10 über eine proximale Hülse 23 mit einem distalen Ende des glockenförmigen Körpers 11 verbunden ist.

Im Folgenden wird die Vorrichtung anhand eines fünften Ausführungsbeispiels beschrieben (Figur 6).

Sofern nichts anderes beschrieben ist, entspricht diese Vorrichtung dem vorstehend beschriebenen Vorrichtungen, insbesondere ist sie ähnlich dem vierten Ausführungsbeispiel ausgebildet. Gleiche Teile sind mit den gleichen Bezugszeichen versehen.

Gemäß dem fünften Ausführungsbeispiel ist vorgesehen, dass alle fünf Körper einzeln bzw. separat mit einem durchgehenden Verbindungsdraht 16 verbunden sind.

Das bedeutet, der erste distale Körper 7 ist spindelförmig ausgebildet und ist mit einer distalen ortsfesten Hülse und einer proximalen verschiebbaren Hülse mit dem Verbindungsdraht verbunden.

Das distale Ende des glockenförmigen zweiten Körpers 8 ist an seinem proximalen Ende mit einer proximalen Hülse ortsfest mit dem Verbindungsdraht 16 verbunden. Der dritte Körper ist an seinem proximalen Ende ortsfest mit dem Verbindungsdraht 16 verbunden und die proximale Hülse des dritten Körpers 9 ist verschiebbar mit dem Verbindungsdraht verbunden. Gleiches gilt für den vierten Körper 10.

Der fünfte, glockenförmig ausgebildete Körper 11 ist entsprechend dem zweiten Körper 8 mit dem Verbindungsdraht verbunden.

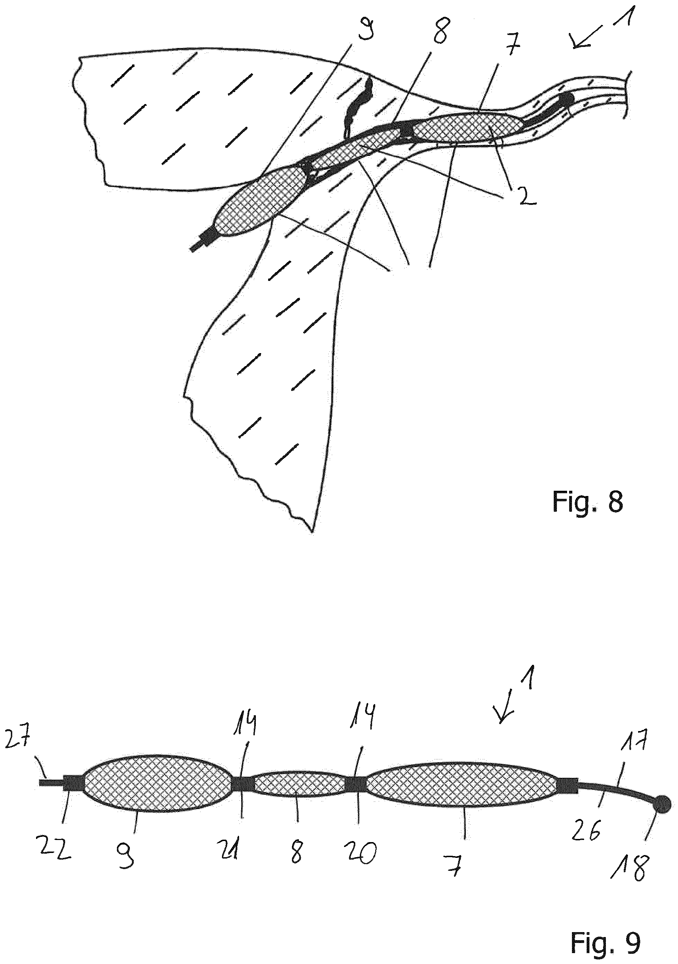

Eine erste Ausführungsform der Uterotubar-Implantat-Vorrichtung 1 umfasst drei komprimierbare selbstexpandierbare Körper 2, die aus einer geflochtenen Gitterstruktur 3 ausgebildet sind. Sofern nichts anderes beschrieben ist kann das bevorzugte Ausführungsbeispiel sämtliche Merkmale der vorstehend erläuterten Ausführungsbeispiele umfassen (Figuren 8 und 9).

Die Körper 2 sind in einer Längsrichtung 4 miteinander derart gekoppelt, dass einer der Körper 2 in Längsrichtung distal 5, der andere Körper 2 in Längsrichtung 4 proximal 6 und der dritte Körper in etwa mittig zwischen diesen beiden angeordnet ist.

Gemäß diesem bevorzugten Ausführungsbeispiel umfasst die Vorrichtung 1 in Längsrichtung 4 von distal 5 nach proximal 6 einen ersten Körper 7, der spindelförmig ausgebildet ist und zur Implantation im isthmischen Abschnitt vorgesehen ist, einen zweiten Körper 8, der spindelförmig ausgebildet ist und zur Implantation im intramuralen Abschnitt vorgesehen ist und einen proximalen Körper 9, der ebenfalls spindelförmig ausgebildet ist und zur Implantation im Uteruscavum vorgesehen ist.

Die Körper 7, 8 und 9 weisen im expandierten Zustand eine vorbestimmte radiale Expansionskraft auf. Diese Expansionskraft ist groß genug bzw. derart bemessen, dass die Körper einen Eileiterabschnitt, den sie kontaktieren, geringfügig aufdehnen können, um einen vollumfänglichen Kontakt der Körper zur Eileiterwandung zu bewirken. Dieser Kontakt wirkt als mechanischer Stimulus für ein Einwachsen der Körper in die Eileiterwand. Zudem muss die radiale Expansionskraft derart bemessen sein, dass ein Kontaktabschnitt 12 (Figur 8) einer Mantelwandung der Körper 13 die Eileiterwandung derart kontaktiert, dass keine als schmerzhaft empfundene chronische Reizung oder gar Penetration der Eileiterwandung auftritt. Das bedeutet, die Expansionskraft muss derart bemessen sein, dass der Kontaktabschnitt 12 der Mantelwandung 13 der Körper atraumatisch mit dem entsprechenden Abschnitt der Eileitennrandung kontaktiert.

Die Körper 7, 8 und 9 sind aus mehreren Drähten derart geflochten, dass eine geflochtene tubuläre Endlos-Struktur entsteht. Diese Struktur ist entsprechend einer Gesamtlänge der Vorrichtung 1 von in etwa 35 mm bis 45 mm an dem proximalen und dem distalen Ende 5, 6 abgelängt.

Die Körper 7, 8 und 9 weisen im expandierten Zustand in den Bereichen Ihrer größten Erstreckung quer zur Längsrichtung der Vorrichtung einen Durchmesser von in etwa 1 bis 3 mm auf. Insbesondere weisen der ersten distale Körper 7 und der dritte proximale Körper 9 einen größeren Durchmesser als der mittlere Körper 8 auf, wobei vorzugsweise der proximale Körper 9 einen größeren Durchmesse aufweist als der distale Körper 7.

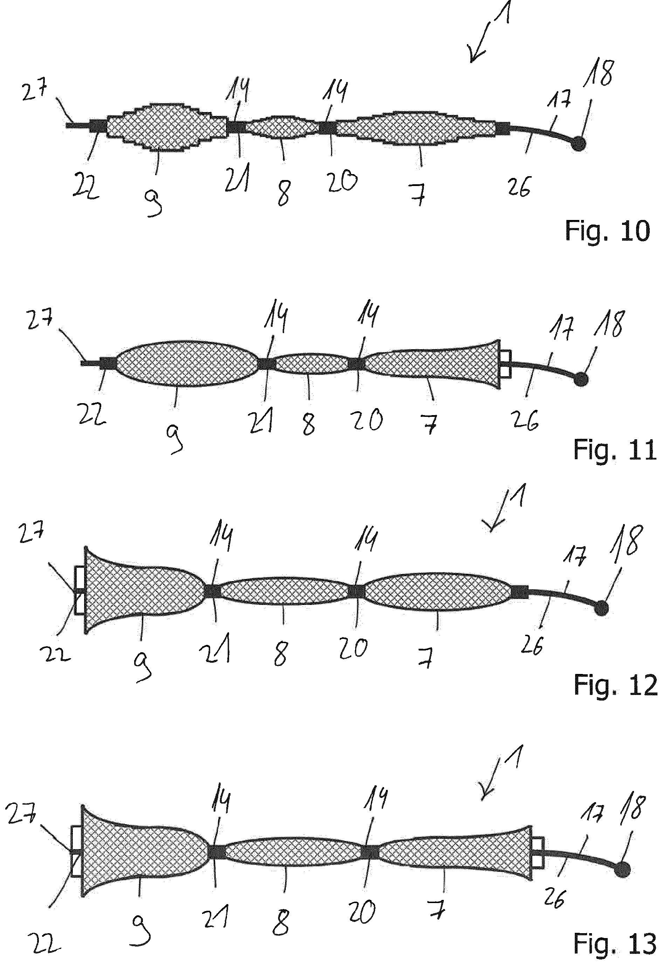

Figur 10 zeigt eine solche Vorrichtung deren Gitterstruktur mittels Laserschneiden hergestellt wurde. Die spindelförmigen Körper weisen im lasergeschnitten Zustand ebenfalls eine Spindelform auf. Diese umfasst jedoch herstellungsbedingt Stufen. Diese Stufen haben eine mechanische Sperrwirkung im Eileiter, so dass bei lasergeschnittener Herstellung die Verwendung trichterförmiger Körper nicht zwingend erforderlich ist, um eine Sperrwirkung zu erzielen.

Gemäß dem bevorzugten vorstehend erläuterten Ausführungsbeispiel umfasst die erfindungsgemäße Vorrichtung drei Körper, wobei alle drei Körper spindelförmig ausgebildet sind (Figuren 8, 9 und 10).

Gemäß einem alternativen Ausführungsbeispiel, wobei die Anordnung der Körper im Eileiter entsprechend dem anhand der Figuren 8 und 9 erläuterten Ausführungsbeispiel entspricht, sind der dritte proximale und der mittlere zweite Körper 8, 9 spindelförmig und der erste distale Körper 7 trichterförmig ausgebildet (Figur 11). Dabei weist vorzugsweise der proximale, erste Körper 9 einen größeren Durchmesser als der distale Körper 7 und der distale, erste Körper 7 eine größeren Durchmesser als der mittlere, zweite Körper 8 auf.

Alternativ können auch der mittlere, zweite und der distale Körper 8, 7 spindelförmig und der proximale, dritte Körper 9 trichterförmig ausgebildet sein (Figur 12).

Weiterhin bevorzugt kann auch vorgesehen sein, dass der dritte, proximale und der erste distale Körper 9, 7 trichterförmig und der mittlere zweite Körper 8 spindelförmig ausgebildet sind. Der proximale Körper kann dann von distal nach proximal oder von proximal nach distal konisch aufweitend ausgebildet sein Der distale Körper kann dann von distal nach proximal oder von proximal nach distal konisch aufweitend ausgebildet sein. Bevorzugt ist jedoch, dass gemäß einer solchen Ausbildung der Vorrichtung der proximale Körper von distal nach proximal und der distale Körper von proximal nach distal konisch aufweitend ausgebildet sind (Figur 13). Gemäß einer solchen Ausbildung der Vorrichtung werden der distale, erste und der proximale, dritte trichterförmige Körper als "Sperrtrichtern" bezeichnet.

Im Folgenden werden Alternativen und Details der erfindungsgemäßen Vorrichtung beschrieben.

Figur 7 ist eine Detailansicht eines Endes eines trichterförmigen Körpers der erfindungsgemäßen Vorrichtung. Hierbei ist vorgesehen, dass die proximalen Enden zu Schlaufen geformt und daher weniger traumatisch ausgebildet sind.

Die Körper gemäß dem ersten und dem zweiten Ausführungsbeispiel sind aus einer tubulären Endlosstruktur ausgebildet, wobei die Kopplungsabschnitte durch radiale Kompression hergestellt sind. Die Einschnürungen können durch Schweißpunkte, Kleben oder eben die vorstehend beschriebenen Hülsen erfolgen.

Hierbei ist dann vorgesehen, dass lediglich eine Sondenspitze mit einer atraumatischen Kugel an einem distalen Ende des distalen Körpers vorgesehen ist.

Ein Abschnitt zwischen zwei Körpern und/oder ein zwischen zwei Körpern angeordneter Abschnitt des Verbindungsdrahtes, über den die Körper miteinander gekoppelt sind, wird als Kopplungsabschnitt bezeichnet, wobei der Kopplungsabschnitt derart flexibel ausgebildet ist, dass die Körper an einen anatomischen Verlauf eines Eileiters anpassbar sind.

Gemäß einer alternativen Ausführungsform können die Körper aus einem einzelnen Draht geflochten sein.

Gemäß einer weiteren Alternative können die Körper aus einem rohrförmigen Körper mittels Laserschneiden hergestellt werden.

Beim Laserschneiden kann die radiale Expansionskraft durch die folgenden Parameter eingestellt werden: durch das Schnittmuster des Laserschnitt-Gitters und durch Durchmesser, Dicke und Material des Rohr-Halbzeugs und dessen Formgebung bei Temperung, wobei vorzugsweise eine Formgedächtnis-Legierung wie z.B. Nitinol eingesetzt wird.

Der Verbindungsdraht 16 kann alternativ auch aus mehreren Drähten und somit strangförmig oder aus mehreren miteinander verwundenen Drähten und somit wendelförmig ausgebildet sein. Gemäß einer weiteren Ausführungsform kann der Verbindungsdraht 16 einen äußeren spiralförmigen Draht umfassen, der einen zentralen Draht umfasst. Dieser zentrale Draht bildet eine Seele aus, um die Zugkräfte die auf den Verbindungsdraht wirken aufzunehmen.

Beim Vorsehen eines Verbindungsdrahtes müssen die Körper derart ausreichend voneinander beabstandet sein bzw. ihre distalen und proximalen Hülsen müssen ausreichend voneinander beabstandet sein, um die Verkürzung beim Expandieren derart mitzumachen, dass sie einander nicht kontaktieren

Gemäß allen vorstehend beschriebenen Ausführungsformen kann ein proximaler Ausstoss-Drahtabschnitt 27 vorgesehen sein, der jedoch keine Verbindungsfunktion zwischen den Körpern übernimmt, sondern der besseren Ausstoßbarkeit des in einem Katheter angeordneten komprimierten Implantats dient.

Grundsätzlich muss die Vorrichtung bzw. müssen die einzelnen Körper derart ausgebildet sein, dass nachdem die Vorrichtung am Implantationsort im Eileiter angeordnet worden ist und der Katheter, der zum Einbringen verwendet wurde, zurückgezogen ist, die Körper in Längsrichtung genügend Bewegungsfreiraum haben, um ihren vollständig expandierten Zustand einzunehmen.

Alternativ zu einer Selbstexpansion können die mehreren Körper mittels einem oder mehreren Ballonkatheter(n) nacheinander oder gleichzeitig expandiert werden.

Fig. 10 zeigt eine perspektivische Ansicht eines mittels Laserschneiden erzeugten Schnittmusters eines Verbindungsbereiches zweier Körper der Vorrichtung in einer seitlichen Ansicht.

Bei Herstellung der Körper durch Laserschnitt können bei radialer Entfaltung insbesondere an den Durchmesserübergängen Stufen durch die Gitterstruktur der Körper entstehen, wie z.B. am proximalen und am distalen Ende eines Körpers und/oder auch im Bereich dazwischen. Solche Stufen sind dahingehend vorteilhaft, da sie in Kontakt mit der Eileiterwand eine Verankerung und Sperrwirkung gegen Dislokation im Eileiter ausüben.

### Bezugszeichenliste

- 1.: Uterotubar-Implantat-Vorrichtung
- 2.: Körper
- 3.: Gitterstruktur
- 4.: Längsrichtung
- 5.: distal
- 6.: proximal
- 7.: erster Körper
- 8.: zweiter Körper
- 9.: dritter Körper
- 10.: vierter Körper
- 11.: fünfter Körper
- 12.: Kontaktabschnitt
- 13.: Mantelwandung
- 14.: Kopplungsabschnitt
- 15.: Hülse
- 16.: Verbindungsdraht
- 17.: Sondenspitze
- 18.: atraumatische Kugel
- 19.: distale Hülse
- 20.: Hülse
- 21.: Hülse
- 22.: Hülse
- 23.: Hülse
- 24.: Hülse
- 25.: Verbindungsdrahtabschnitt
- 26: distaler Drahtabschnitt
- 27: proximaler Ausstoßabschnitt

## Patentansprüche

1. Uterotubar-Implantat-Vorrichtung (1) umfassend
zumindest drei komprimierbare selbstexpandierbare Körper (2), wobei die Körper (2) aus einer Gitterstruktur (3) ausgebildet sind und in einer Längsrichtung (4) miteinander gekoppelt sind, wobei ein Körper (2) der in Längsrichtung (4) distal (5) angeordnet ist und ein Körper (2) der in Längsrichtung (4) proximal (6) angeordnet ist, derart trichterförmig ausgebildet sind, dass der proximale (6) trichterförmige Körper (2) in Längsrichtung (4) von distal (5) nach proximal (6) und der distale Körper (5, 2) von proximal (6) nach distal (5) konisch aufweitend ausgebildet ist, und ein zwischen dem distalen trichterförmigen Körper (5, 2) und dem proximalen trichterförmigen Körper (6, 2) angeordneter Körper (2) derart spindelförmig ausgebildet ist, dass der spindelförmige Körper (2) in Längsrichtung (4) an seinem proximalen (6) Ende derart konisch aufweitend und an seinem distalen (5) Ende derart konisch verjüngend ausgebildet ist, dass der Körper (2) eine Spindelform ausbildet.

2. Uterotubar-Implantat-Vorrichtung (1) nach Anspruch 1,
**dadurch gekennzeichnet,**
**dass** die Körper (2) im expandierten Zustand unterschiedliche Durchmesser aufweisen, wobei der oder die mittleren Körper (2) im expandierten Zustand einen geringeren Durchmesser aufweisen als der proximale (6) und der distale (5) Körper (2).

3. Uterotubar-Implantat-Vorrichtung (1) nach einem der Ansprüche 1 oder 2,
**dadurch gekennzeichnet,**
**dass** ein Verbindungsdraht (16) oder Verbindungsdrahtabschnitte (25) vorgesehen sind, die sich durch die Körper (2) in Längsrichtung (4) hindurcherstrecken, wobei die Körper (2) zumindest an ihren proximalen (6) und/oder distalen (5) Enden mit dem Verbindungsdraht (16) oder den Verbindungsdrahtabschnitten (25) derart verbunden sind, dass die Kopplung der Körper (2) mittels des Verbindungsdrahtes (16) erfolgt, wobei der Verbindungsdraht (16) oder die Verbindungsdrahtabschnitte (25) aus einem oder mehreren Drähten aus Metall oder Kunststoff strang- oder wendelförmig aus Metall oder Kunststoff ausgebildet ist.

4. Uterotubar-Implantat-Vorrichtung (1) nach Anspruch 3,
**dadurch gekennzeichnet,**
**dass** am distalen (5) Ende des distalen (5) Körpers (2) ein distaler Drahtabschnitt (26) oder am distalen (5) Ende des Verbindungsdrahtes (16) oder eines Verbindungsdrahtabschnittes (25) eine Sondenspitze (17) vorgesehen ist.

5. Uterotubar-Implantat-Vorrichtung (1) nach Anspruch 3,
**dadurch gekennzeichnet,**
**dass** ein proximales (6) Ende des spindelförmigen Körpers (2), mittels einer Hülse (15), mit dem Verbindungsdraht (16) derart gekoppelt ist, dass das proximale (6) Ende dieses Körpers (2) in Längsrichtung (4) verschiebbar mit dem Verbindungsdraht (16) verbunden ist, und ein distales (5) Ende des spindelförmigen Körpers, mittels einer Hülse (15), fest mit dem Verbindungsdraht (16) verbunden ist.

6. Uterotubar-Implantat-Vorrichtung (1) nach einem der Ansprüche 3 bis 5,
**dadurch gekennzeichnet,**
**dass** ein Abschnitt der Körper (2) und/oder ein zwischen zwei Körpern (2) angeordneter Abschnitt des Verbindungsdrahtes (16), über den die Körper (2) miteinander gekoppelt sind, als Kopplungsabschnitt (14) bezeichnet wird, wobei der Kopplungsabschnitt (14) derart flexibel ausgebildet ist, dass die Körper (2) an einen anatomischen Verlauf eines Eileiters anpassbar sind.

7. Uterotubar-Implantat-Vorrichtung (1) nach einem der Ansprüche 1 bis 6,
**dadurch gekennzeichnet,**
**dass** die Länge der Vorrichtung vom distalen (5) Ende des distalen Körpers (5, 2) bis zum proximalen (6) Ende des proximalen Körpers (6, 2) 35 mm bis 45 mm, und der Durchmesser im expandierten Zustand 1 mm bis 4 mm beträgt.

8. Uterotubar-Implantat-Vorrichtung (1) nach einem der Ansprüche 1 bis 7,
**dadurch gekennzeichnet,**
**dass** die Körper (2) zumindest teilweise einen unterschiedlichen Durchmesser aufweisen und ein Körper, der im Uteruskavum bzw. Uterotubarwinkel anordbar ist, als distaler Körper (5, 2) bezeichnet wird, der einen Durchmesser von 3 mm bis 4 mm aufweist, und ein oder zwei Körper (2) zur Implantation im intramuralen Abschnitt vorgesehen sind und der oder die Körper (2) als zweiter und dritter Körper (8, 9) bezeichnet werden, wobei der oder die Körper (2) einen Durchmesser von 2 mm aufweisen und ein Körper (2) zur Implantation im isthmischen Abschnitt vorgesehen ist, wobei dieser Körper (2) als proximaler Körper (6, 2) bezeichnet wird, wobei der proximale Körper (6, 2) einen Durchmesser von 3 bis 4 mm aufweist.

9. Uterotubar-Implantat-Vorrichtung (1) nach einem der Ansprüche 1 bis 8,
**dadurch gekennzeichnet,**
**dass** das der proximale und der distale Körper (6, 5, 2) im expandierten Zustand einen größeren Durchmesser und eine größere radiale Expansionskraft aufweisen als zumindest ein dazwischen angeordneter mittlerer Körper (2).

10. Uterotubar-Implantat-Vorrichtung (1) nach einem der Ansprüche 1 bis 9,
**dadurch gekennzeichnet,**
**dass** die Körper (2) aus einem oder mehreren Drähten geflochten oder aus einem rohrförmigen Körper (2) mittels Laserschneiden hergestellt sind, wobei die Öffnungen der Körper (2) eine Öffnungsweite von kleiner 1 mm oder von 300 µm bis 600 µm aufweisen.

11. Uterotubar-Implantat-Vorrichtung (1) nach einem der Ansprüche 1 bis 10,
**dadurch gekennzeichnet,**
**dass** die Körper (2) im expandierten Zustand eine vorbestimmte radiale Expansionskraft aufweisen, die groß genug ist, den Eileiterabschnitt aufzudehnen, um einen Kontaktabschnitt (12) einer Mantelwandung (13) der Körper (2) zur Eileiterwand auszubilden, wobei der Kontaktabschnitt (12) derart mit der Eileiterwandung kontaktiert, dass ein mechanischer Stimulus derart erzeugt wird, dass zumindest ein Bereich des Kontaktabschnittes (12) der Körper mit der Eileiterwandung verwächst, und die entsprechende Gitterstruktur (3) derart atraumatisch den Eileiterabschnitt kontaktiert, dass die Eileiterwand nicht beschädigt wird.

12. Uterotubar-Implantat-Vorrichtung (1) nach einem der Ansprüche 1 bis 11,
**dadurch gekennzeichnet,**
**dass** die Mantelflächen eines oder mehrerer Körper (2) ganz oder zumindest teilweise beschichtet sind, wobei die Beschichtung eine oder mehrere Komponenten umfassen kann, bestehend aus einem Polymer oder einem quellbaren Hydrogel und/oder mit einem bioaktiven Wirkstoff dotiert ist und/oder dieser Wirkstoff ein Spermizid und/oder ein Anti-Progesteron ist, wodurch die Blutungsneigung reduziert und ein Einwachsen des Implantats gefördert wird.

13. Uterotubar-Implantat-Vorrichtung (1) nach Anspruch 12,
**dadurch gekennzeichnet,**
**dass** das Polymer Polyurethan oder ein bioabbaubare Polylactid ist und/oder die Beschichtung eine Hydrogelkomponente beinhaltet und/oder dass das Spermizid Nonoxinol 9 ist.

14. Uterotubar-Implantat-Vorrichtung (1) nach einem der Ansprüche 1 bis 13,
**dadurch gekennzeichnet,**
**dass** der spindelförmige Körper mit einem verschlussfördernden Material gefüllt ist und/oder wobei die Fasern mit Hydrogel imprägniert sind und/oder dass Fasern zur Erhöhung der spermiziden Wirkung eingewoben oder eingeflochten sind.

15. Uterotubar-Implantat-Vorrichtung (1) nach Anspruch 14,
**dadurch gekennzeichnet,**
**dass** das verschlussfördernden Material biokompatible Textilfasern aus Polyethylenterphtalat umfasst und/oder gefüllt ist und/oder
wobei die Fasern mit Hydrogel imprägniert sind und/oder dass die Fasern zur Erhöhung der spermiziden Wirkung Kupferfäden sind.

## Claims

1. A uterotubal implant device (1), comprising
at least three compressible self-expanding bodies (2), the bodies (2) being constituted by a reticular structure (3) and coupled to one another in a longitudinal direction (4), a body (2) which is arranged distally (5) in the longitudinal direction (4) and a body (2) which is arranged proximally (6) in the longitudinal direction (4) being formed to be funnel-shaped in such a way that the proximal (6) funnel-shaped body (2) in the longitudinal direction (4) is formed to be conically widening from distal (5) to proximal (6) and the distal body (5, 2) is formed to be conically widening from proximal (6) to distal (5), and a body (2) arranged between the distal funnel-shaped body (5, 2) and the proximal funnel-shaped body (6, 2) being formed to be spindle-shaped in such a way that the spindle-shaped body (2) is formed in the longitudinal direction (4) to be conically widening at its proximal (6) end and conically tapering at its distal (5) end in such a way that the body (2) forms a spindle shape.

2. The uterotubal implant device (1) according to claim 1,
**characterized in that**
the bodies (2) have different diameters in the expanded state, with the medial body or bodies (2) having a smaller diameter in the expanded state than the proximal (6) and distal (5) bodies (2).

3. The uterotubal implant device (1) according to any of claims 1 or 2,
**characterized in that**
a connecting wire (16) or connecting wire sections (25) extending through the bodies (2) in the longitudinal direction (4) are provided, the bodies (2) are connected to the connecting wire (16) or the connecting wire sections (25), at least at their proximal (6) and/or distal (5) ends, in such a way that the coupling of the bodies (2) is effected by means of the connecting wire (16), the connecting wire (16) or the connecting wire sections (25) being formed from metal or plastic in the shape of a strand or spiral made of metal or plastic.

4. The uterotubal implant device (1) according to claim 3,
**characterized in that**
a distal wire section (26) is provided at the distal (5) end of the distal (5) body (2) or a probe tip (17) is provided at the distal (5) end of the connecting wire (16) or a connecting wire section (25).

5. The uterotubal implant device (1) according to claim 3,
**characterized in that**
a proximal (6) end of the spindle-shaped body (2) is coupled to the connecting wire (16) by means of a sleeve (15) in such a way that the proximal (6) end of said body (2) is connected to the connecting wire (16) to be slidable in the longitudinal direction (4), and a distal (5) end of the spindle-shaped body is fixedly connected to the connecting wire (16) by means of a sleeve (15).

6. The uterotubal implant device (1) according to any of claims 3 to 5,
**characterized in that**
a section of the bodies (2) and/or a section of the connecting wire (16) arranged between the bodies (2), via which the bodies (2) are coupled to one another, is designated as a coupling section (14), the coupling section (14) being designed to be flexible in such a way that the bodies (2) are adaptable to an anatomical course of a fallopian tube.

7. The uterotubal implant device (1) according to any of claims 1 to 6,
**characterized in that**
the length of the device from the distal (5) end of the distal body (5, 2) to the proximal (6) end of the proximal body (6, 2) is 35 mm to 45 mm, and the diameter in the expanded state is 1 mm to 4 mm.

8. The uterotubal implant device (1) according to any of claims 1 to 7,
**characterized in that**
the bodies (2) have at least in part different diameters and a body which is disposable in the uterine cavity or in a uterotubal corner is designated as the distal body (5, 2) which has a diameter of 3 mm to 4 mm, and one or two bodies (2) are provided for implantation in the intramural section and the body or bodies (2) are designated as second and third bodies (8, 9), the body or bodies (2) having a diameter of 2 mm and one body (2) being provided for implantation in the isthmic section, said body (2) being designated as the proximal body (6, 2) and the proximal body (6, 2) having a diameter of 3 to 4 mm.

9. The uterotubal implant device (1) according to any of claims 1 to 8,
**characterized in that**
in the expanded state, the proximal and distal bodies (6, 5, 2) have a larger diameter and a greater radial expansion force than at least one medial body (2) arranged therebetween.

10. The uterotubal implant device (1) according to any of claims 1 to 9,
**characterized in that**
the bodies (2) are woven from one or more wires or manufactured from a tubular body (2) by laser cutting, the openings of the bodies (2) having an opening width of less than 1 mm or from 300 µm to 600 µm.

11. The uterotubal implant device (1) according to any of claims 1 to 10,
**characterized in that**
the bodies (2) in the expanded state have a predetermined radial expansion force which is sufficiently great to stretch the fallopian tube section in order to form a contact section (12) of an envelope wall (13) of the bodies (2) with the fallopian tube wall, the contact section (12) being in contact with the fallopian tube wall in such a way that a mechanical stimulus is generated in such a way that at least one area of the contact section (12) of the bodies grows together with the fallopian tube wall, and the corresponding reticular structure (3) is in contact with the fallopian tube section in an atraumatic manner in such a way that the fallopian tube wall is not damaged.

12. The uterotubal implant device (1) according to any of claims 1 to 11,
**characterized in that**
the envelope surfaces of one or more bodies (2) are wholly or at least partly coated, with the option that the coating comprises one or more components consisting of a polymer or a swellable hydrogel and/or is provided with a bioactive agent and/or said agent is a spermicide and/or an anti-progesterone, whereby the bleeding tendency is reduced and engraftment of the implant is promoted.

13. The uterotubal implant device (1) according to claim 12,
**characterized in that**
the polymer is polyurethane or biodegradable polylactide and/or the coating contains a hydrogel component and/or the spermicide is Nonoxynol 9.

14. The uterotubal implant device (1) according to any of claims 1 to 13,
**characterized in that**
the spindle-shaped body is filled with an occlusion-promoting material and/or the fibers are impregnated with hydrogel and/or fibers are interwoven or interlaced to increase the spermicidal effect.

15. The uterotubal implant device (1) according to claim 14,
**characterized in that**
the occlusion-promoting material comprises and/or is filled with bio-compatible textile fibers made of polyethylene terephthalate and/or
the fibers are impregnated with hydrogel and/or
the fibers are copper wires for increasing the spermicidal effect.

## Revendications

1. Dispositif d'implant utéro-tubaire (1) comprenant
au moins trois corps (2) compressibles auto-expansibles, sachant que les corps (2) sont constitués par une structure réticulaire (3) et sont couplés les uns aux autres dans une direction longitudinale (4), sachant qu'un corps (2) qui est disposé distalement (5) en direction longitudinale (4) et un corps (2) qui est disposé proximalement (6) en direction longitudinale (4) sont constitués en forme d'entonnoir de telle manière que le corps (2) proximal (6) en forme d'entonnoir soit constitué en direction longitudinale (4) en s'élargissant coniquement (4) de distal (5) à proximal (6) et le corps distal (5, 2) en s'élargissant coniquement de proximal (6) à distal (5), et un corps (2) disposé entre le corps distal (5, 2) en forme d'entonnoir et le corps proximal (6, 2) en forme d'entonnoir est constitué en forme de fuseau de telle manière que le corps (2) en forme de fuseau soit constitué en direction longitudinale (4) en s'élargissant coniquement à son extrémité proximale (6) et en s'amincissant coniquement à son extrémité distale (5) de telle manière que le corps (2) constitue une forme de fuseau.

2. Dispositif d'implant utéro-tubaire (1) selon la revendication 1,
**caractérisé en ce que**
les corps (2) présentent à l'état expansé des diamètres différents, sachant que le ou les corps (2) médians présentent à l'état expansé un diamètre plus petit que le corps (2) proximal (6) et le corps (2) distal (5).

3. Dispositif d'implant utéro-tubaire (1) selon l'une des revendications 1 ou 2,
**caractérisé en ce que**
un fil de liaison (16) ou des sections de fil de liaison (25) qui s'étendent à travers les corps (2) en direction longitudinale (4) sont prévus, sachant que les corps (2) sont reliés au fil de liaison (16) ou aux sections de fil de liaison (25), du moins à leurs extrémités proximales (6) et/ou distales (5), de telle manière que le couplage des corps (2) soit effectué moyennant le fil de liaison (16), sachant que le fil de liaison (16) ou les sections de fil de liaison (25) sont constituées en forme de cordon ou de spirale en métal ou en matière plastique par un ou plusieurs fils en métal ou en matière plastique.

4. Dispositif d'implant utéro-tubaire (1) selon la revendication 3,
**caractérisé en ce que**
une section de fil distale (26) est prévue à l'extrémité distale (5) du corps (2) distal (5) ou une pointe de sonde (17) est prévue à l'extrémité distale (5) du fil de liaison (16) ou d'une section de fil de liaison (25).

5. Dispositif d'implant utéro-tubaire (1) selon la revendication 3,
**caractérisé en ce que**
une extrémité proximale (6) du corps (2) en forme de fuseau est couplée au fil de liaison (16), moyennant un manchon (15), de telle manière que l'extrémité proximale (6) de ce corps (2) soit reliée en direction longitudinale (4) au fil de liaison (16) de manière coulissante, et une extrémité distale (5) du corps en forme de fuseau soit reliée de manière fixe au fil de liaison (16), moyennant un manchon (15).

6. Dispositif d'implant utéro-tubaire (1) selon l'une des revendications 3 à 5,
**caractérisé en ce que**
une section des corps (2) et/ou une section du fil de liaison (16) disposée entre les corps (2) via laquelle les corps (2) sont couplés les uns aux autres est désignée comme section de couplage (14), sachant que la section de couplage (14) est constituée de manière flexible de telle manière que les corps (2) soient adaptables à un tracé anatomique d'une trompe.

7. Dispositif d'implant utéro-tubaire (1) selon l'une des revendications 1 à 6,
**caractérisé en ce que**
la longueur du dispositif depuis l'extrémité distale (5) du corps distal (5, 2) jusqu'à l'extrémité proximale (6) du corps proximal (6, 2) est de 35 mm à 45 mm, et le diamètre à l'état expansé est de 1 mm à 4 mm.

8. Dispositif d'implant utéro-tubaire (1) selon l'une des revendications 1 à 7,
**caractérisé en ce que**
les corps (2) présentent au moins en partie un diamètre différent et un corps qui est disposable dans la cavité de l'utérus ou dans un angle utéro-tubaire est désigné comme corps distal (5, 2) qui présente un diamètre de 3 mm à 4 mm, et un ou deux corps (2) sont prévus pour l'implantation dans la section intramurale et le ou les corps (2) sont désignés comme deuxième et troisième corps (8, 9), sachant que le ou les corps (2) présentent un diamètre de 2 mm et un corps (2) est prévu pour l'implantation dans la section isthmique, sachant que ce corps (2) est désigné comme corps proximal (6, 2), sachant que le corps proximal (6, 2) présente un diamètre de 3 à 4 mm.

9. Dispositif d'implant utéro-tubaire (1) selon l'une des revendications 1 à 8,
**caractérisé en ce que**
les corps proximal et distal (6, 5, 2) présentent à l'état expansé un diamètre plus grand et une force d'expansion radiale plus grande qu'au moins un corps (2) médian disposé entre ceux-ci.

10. Dispositif d'implant utéro-tubaire (1) selon l'une des revendications 1 à 9,
**caractérisé en ce que**
les corps (2) sont tissés à partir d'un ou de plusieurs fils ou fabriqués à partir d'un corps (2) tubulaire par découpe laser, sachant que les ouvertures des corps (2) présentent une largeur d'ouverture inférieure à 1 mm ou de 300 µm à 600 µm.

11. Dispositif d'implant utéro-tubaire (1) selon l'une des revendications 1 à 10,
**caractérisé en ce que**
les corps (2) présentent à l'état expansé une force d'expansion radiale prédéterminée qui est suffisamment grande pour étirer la section de trompe afin de constituer une section de contact (12) d'une paroi d'enveloppe (13) des corps (2) avec la paroi de trompe, sachant que la section de contact (12) est en contact avec la paroi de trompe de telle manière qu'un stimulus mécanique soit généré de telle manière qu'au moins une zone de la section de contact (12) des corps fusionne avec la paroi de trompe, et la structure réticulaire (3) correspondante est en contact avec la section de trompe de manière atraumatique telle que la paroi de trompe ne soit pas endommagée.

12. Dispositif d'implant utéro-tubaire (1) selon l'une des revendications 1 à 11,
**caractérisé en ce que**
les surfaces d'enveloppe d'un ou de plusieurs corps (2) sont en tout ou au moins en partie revêtues, sachant que le revêtement peut comprendre un ou plusieurs composants constitués par un polymère et un hydrogel gonflable et/ou est doté d'un agent bioactif et/ou cet agent est un spermicide et/ou un anti-progestérone, ce par quoi la tendance au saignement est réduite et un ancrage de l'implant est favorisé.

13. Dispositif d'implant utéro-tubaire (1) selon la revendication 12,
**caractérisé en ce que**
le polymère est du polyuréthane ou un polylactide biodégradable et/ou le revêtement contient un composant d'hydrogel et/ou le spermicide est du Nonoxinol 9.

14. Dispositif d'implant utéro-tubaire (1) selon l'une des revendications 1 à 13,
**caractérisé en ce que**
le corps en forme de fuseau est rempli d'un matériau favorisant l'occlusion et/ou sachant que les fibres sont imprégnées d'hydrogel et/ou des fibres sont tissées ou tressées pour augmenter l'effet spermicide.

15. Dispositif d'implant utéro-tubaire (1) selon la revendication 14,
**caractérisé en ce que**
le matériau favorisant l'occlusion comprend et/ou est rempli de fibres textiles biocompatibles en téréphtalate de polyéthylène et/ou
les fibres sont imprégnées d'hydrogel et/ou
les fibres destinées à augmenter l'effet spermicide sont des fils de cuivre.
